(19) Europäisches Patentamt
European Patent Office
Office européen des brevets

(11) EP 1 075 477 B1

(12) EUROPÄISCHE PATENTSCHRIFT

(45) Veröffentlichungstag und Bekanntmachung des Hinweises auf die Patenterteilung:
**12.03.2003 Patentblatt 2003/11**

(21) Anmeldenummer: **99922133.6**

(22) Anmeldetag: **27.04.1999**

(51) Int Cl.[7]: **C07D 471/04**, A61K 31/44, C07D 491/14
// (C07D471/04, 221:00), C07D221:00

(86) Internationale Anmeldenummer:
**PCT/EP99/02827**

(87) Internationale Veröffentlichungsnummer:
**WO 99/057118 (11.11.1999 Gazette 1999/45)**

(54) **Neue Benzonaphtyridin-N-oxide**

Novel Benzonaphthyridine-N-oxides

Nouveaux N-oxydes de benzonaphtyridine

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **05.05.1998 EP 98108124**

(43) Veröffentlichungstag der Anmeldung:
**14.02.2001 Patentblatt 2001/07**

(73) Patentinhaber: **ALTANA Pharma AG**
**78467 Konstanz (DE)**

(72) Erfinder:
- **GUTTERER, Beate**
  **D-78476 Allensbach (DE)**
- **AMSCHLER, Hermann**
  **D-78315 Radolfzell (DE)**
- **ULRICH, Wolf-Rüdiger**
  **D-78464 Konstanz (DE)**
- **MARTIN, Thomas**
  **D-78462 Konstanz (DE)**
- **BÄR, Thomas**
  **D-78479 Reichenau (DE)**
- **HATZELMANN, Armin**
  **D-78467 Konstanz (DE)**
- **BOSS, Hildegard**
  **D-78476 Allensbach (DE)**
- **BEUME, Rolf**
  **D-78465 Konstanz (DE)**
- **BUNDSCHUH, Daniela**
  **D-78462 Konstanz (DE)**
- **KLEY, Hans-Peter**
  **D-78476 Allensbach (DE)**
- **FLOCKERZI, Dieter**
  **D-78476 Allensbach (DE)**

(56) Entgegenhaltungen:
**EP-A- 0 247 971**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 075 477 B1

## Beschreibung

Die Erfindung betrifft neue Benzonaphthyridin-N-Oxide, die In der pharmazeutischen Industrie zur Herstellung von Medikamenten verwendet werden.

In der DE-OS 21 23 328 und im USP 3,899,494 werden substituierte Benzonaphthyridine beschrieben, die sich durch ausgeprägte Blutplättchenaggregationshemmung auszeichnen. In EP 247 971 und WO91/17991 werden 6-Phenylbenzonaphthyridine zur Behandlung entzündlicher Atemwegserkrankungen offenbart.

Es wurde nun gefunden, daß die nachfolgend näher beschriebenen Verbindungen der Formel I, die sich von den Verbindungen der EP 247 971 bzw. WO91/17991 durch die Substitution am 6-Phenylring und das Vorhandensein eines N-Oxids in 2-Position unterscheiden, überraschende und besonders vorteilhafte Eigenschaften besitzen.

Gegenstand der Erfindung sind somit Verbindungen der Formel I,

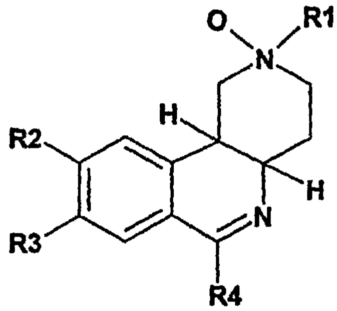

(I)

worin

R1 1-4C-Alkyl bedeutet,
R2 Hydroxy, 1-4C-Alkoxy, 3-7C-Cycloalkoxy, 3-7C-Cycloalkylmethoxy oder ganz oder überwiegend durch Fluor substituiertes 1-4C-Alkoxy bedeutet,
R3 Hydroxy, 1-4C-Alkoxy, 3-7C-Cydoalkoxy, 3-7C-Cycloalkylmethoxy oder ganz oder überwiegend durch Fluor substituiertes 1-4C-Alkoxy bedeutet,

oder worin

R2 und R3 gemeinsam eine 1-2C-Alkylendioxygruppe bedeuten,
R4 einen durch R5 und R6 substituierten Phenylrest darstellt, wobei
R5 Wasserstoff, Hydroxy, Halogen, Nitro, 1-4C-Alkyl, Trifluormethyl oder 1-4C-Alkoxy bedeutet,
R6 CO-R7 oder CO-R8 bedeutet, wobei
R7 Hydroxy, 1-8C-Alkoxy, 3-7C-Cycloalkoxy oder 3-7C-Cycloalkylmethoxy bedeutet und
R8 N(R81)R82 bedeutet, wobei R81 und R82 unabhängig voneinander Wasserstoff, 1-7C-Alkyl, 3-7C-Cycloalkyl oder 3-7C-Cycloalkylmethyl bedeuten, oder wobei R81 und R82 zusammen und unter Einschluß des Stickstoffatoms, an das beide gebunden sind, einen 1-Pyrrolidinyl-, 1-Piperidyl-, 1-Hexahydroazepinyl- oder 4-Morpholinylrest darstellen,

sowie die Salze dieser Verbindungen.

1-4C-Alkyl steht für einen geradkettigen oder verzweigten Alkylrest mit 1 bis 4 Kohlenstoffatomen. Beispielsweise seien genannt der Butyl-, iso-Butyl-, sec.-Butyl-, tert.-Butyl-, Propyl-, Isopropyl- und bevorzugt der Ethyl- und Methylrest.

1-4C-Alkoxy steht für Reste, die neben dem Sauerstoffatom einen geradkettigen oder verzweigten Alkylrest mit 1 bis 4 Kohlenstoffatomen enthalten. Beispielsweise seien genannt der Butoxy-, iso-Butoxy-, sec.-Butoxy-, tert.-Butoxy-, Propoxy-, Isopropoxy- und bevorzugt der Ethoxy- und Methoxyrest.

3-7C-Cycloalkoxy steht für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht für Cyclopropylmethoxy, Cyclobutylmethoxy, Cyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder überwiegend durch Fluor substituiertes 1-4C-Alkoxy seien beispielsweise der 2,2,3,3,3-Pentafluorpropoxy-, der Perfluorethoxy-, der 1,1,2,2-Tetrafluorethoxy-, der 1,2,2-Trifluorethoxy-, der Trifluormethoxy-, insbesondere der 2,2,2-Trifluorethoxy- und bevorzugt der Difluormethoxyrest genannt.

1-2C-Alkylendioxy steht beispielsweise für den Methylendioxy- ($-O-CH_2-O-$) und den Ethylendioxyrest ($-O-CH_2-CH_2-O-$).

Halogen im Sinne der Erfindung ist Fluor, Chlor und Brom.

1-8C-Alkoxy steht für Reste, die neben dem Sauerstoffatom einen geradkettigen oder verzweigten Alkylrest mit 1 bis 8 Kohlenstoffatomen enthalten. Beispielsweise seien genannt der Octyloxy-, Heptyloxy-, Hexyloxy-, Pentyloxy-, Methylbutoxy-, Ethylpropoxy-, Butoxy-, iso-Butoxy-, sec.-Butoxy-, tert.-Butoxy-, Propoxy- und bevorzugt der Isopropoxy-, Ethoxy- und Methoxyrest.

1-7C-Alkyl steht für geradkettige oder verzweigte Alkylreste mit 1 bis 7 Kohlenstoffatomen. Beispielsweise seien genannt der Heptyl-, Isoheptyl- (5-Methylhexyl-), Hexyl-, Isohexyl- (4-Methylpentyl-), Neohexyl- (3,3-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

3-7C-Cycloalkyl steht für den Cyclopropyl-, Cyclobutyl-, Cyclopentyl-, Cyclohexyl- und Cycloheptylrest.

3-7C-Cycloalkylmethyl steht für einen Methylrest, der durch einen der vorstehend genannten 3-7C-Cycloalkylreste substituiert ist. Beispielsweise genannt seien die Cycloalkylmethylreste Cyclopropylmethyl, Cyclobutylmethyl und Cyclopentylmethyl.

Als Salze kommen für Verbindungen der Formel I - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren und Basen. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure. Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen - zum Beispiel im Fall einer Carboxyl-Substitution - auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen-Verbindungen im industriellen Maßstab als Verfahrensprodukte zunächst anfallen können, werden durch dem Fachmann bekannte Verfahren in pharmakologisch verträgliche Salze übergeführt,

Dem Fachmann ist bekannt, daß die erfindungsgemäßen Verbindungen als auch ihre Salze, wenn sie zum Beispiel in kristalliner Form isoliert werden, verschiedene Mengen an Lösungsmitteln enthalten können. Die Erfindung umfaßt daher auch alle Solvate und insbesondere alle Hydrate der Verbindungen der Formel 1, sowie alle Solvate und Insbesondere alle Hydrate der Salze der Verbindungen der Formel I.

Hervorzuhebende Verbindungen der Formel I sind solche, worin

R1 1-4C-Alkyl bedeutet,
R2 1-4C-Alkoxy, 3-7C-Cycloalkoxy, 3-7C-Cycloalkylmethoxy oder ganz oder überwiegend durch Fluor substituiertes 1-2C-Alkoxy bedeutet,
R3 1-4C-Alkoxy, 3-7C-Cycloalkoxy, 3-7C-Cycloalkylmethoxy oder ganz oder überwiegend durch Fluor substituiertes 1-2C-Alkoxy bedeutet,
R4 einen durch R5 und R6 substituierten Phenylrest darstellt, wobei
R5 Wasserstoff, Hydroxy, Halogen, Nitro, 1-4C-Alkyl, Trifluormethyl oder 1-4C-Alkoxy bedeutet,
R6 CO-R7 oder CO-R8 bedeutet, wobei
R7 Hydroxy, 1-8C-Alkoxy, 3-7C-Cycloalkoxy oder 3-7C-Cycloalkylmethoxy bedeutet und
R8 N(R81)R82 bedeutet, wobei R81 und R82 unabhängig voneinander Wasserstoff, 1-70-Alkyl, 3-7C-Cycloalkyl oder 3-7C-Cycloalkylmethyl bedeuten, oder wobei R81 und R82 zusammen und unter Einschluß des Stickstoffatoms, an das beide gebunden sind, einen 1-Piperidyl-, 1-Hexahydroazepinyl- oder 4-Morpholinylrest darstellen,

sowie die Salze dieser Verbindungen.

Eine Ausgestaltung der hervorzuhebenden Verbindungen der Formel I sind solche, worin

R1 Methyl bedeutet,

R2 1-4C-Alkoxy bedeutet,
R3 1-4C-Alkoxy bedeutet,
R4 einen durch R5 und R6 substituierten Phenylrest darstellt, wobei
R5 Wasserstoff, Hydroxy, 1-4C-Alkyl oder 1-4C-Alkoxy bedeutet,
R6 CO-R7 oder CO-R8 bedeutet, wobei
R7 Hydroxy, 1-8C-Alkoxy oder 3-7C-Cycloalkoxy bedeutet und
R8 N(R81)R82 bedeutet, wobei R81 und R82 unabhängig voneinander Wasserstoff, 1-7C-Alkyl oder 3-7C-Cycloalkyl bedeuten, oder wobei R81 und R82 zusammen und unter Einschluß des Stickstoffatoms, an das beide gebunden sind, einen 1-Piperidyl-, 1-Hexahydroazepinyl- oder 4-Morpholinylrest darstellen,

sowie die Salze dieser Verbindungen.

Besonders hervorzuhebende Verbindungen der Formel I sind solche, in denen

R1 Methyl bedeutet,
R2 Methoxy, Ethoxy oder Propoxy bedeutet,
R3 Methoxy oder Ethoxy bedeutet,
R4 einen durch R5 und R6 substituierten Phenylrest darstellt, wobei
R5 Wasserstoff bedeutet,
R6 CO-R7 oder CO-R8 bedeutet, wobei
R7 Hydroxy oder 1-8C-Alkoxy bedeutet und
R8 N(R81)R82 bedeutet, wobei R81 und R82 unabhängig voneinander Wasserstoff oder 1-4C-Alkyl oder 5-7C-Cycloalkyl bedeuten, oder wobei R81 und R82 zusammen und unter Einschluß des Stickstoffatoms, an das beide gebunden sind, einen 1-Piperidyl-, 1-Hexahydroazepinyl- oder 4-Morpholinylrest darstellen,

sowie die Salze dieser Verbindungen.

Eine Ausgestaltung der besonders hervorzuhebenden Verbindungen der Formel I sind solche, in denen

R1 Methyl bedeutet,
R2 Methoxy oder Ethoxy bedeutet,
R3 Methoxy oder Ethoxy bedeutet,
R4 einen durch R5 und R6 substituierten Phenylrest darstellt, wobei
R5 Wasserstoff bedeutet,
R6 CO-R7 oder CO-R8 bedeutet, wobei
R7 Hydroxy oder 1-8C-Alkoxy bedeutet und
R8 N(R81)R82 bedeutet, wobei R81 und R82 unabhängig voneinander Wasserstoff oder 1-4C-Alkyl oder 5-7C-Cycloalkyl bedeuten, oder wobei R81 und R82 zusammen und unter Einschluß des Stickstoffatoms, an das beide gebunden sind, einen 1-Piperidyl-, 1-Hexahydroazepinyl- oder 4-Morpholinylrest darstellen,

sowie die Salze dieser Verbindungen.

Bevorzugte Verbindungen der Formel I sind solche, in denen

R1 Methyl bedeutet,
R2 Ethoxy oder Propoxy bedeutet,
R3 Methoxy oder Ethoxy bedeutet,
R4 einen durch R5 und R6 substitulerten Phenylrest darstellt, wobei
R5 Wasserstoff bedeutet,
R6 CO-R7 oder CO-R8 bedeutet, wobei
R7 1-4C-Alkoxy bedeutet und
R8 N(R81)R82 bedeutet, wobei R81 und R82 unabhängig voneinander 1-4C-Alkyl oder 5-7C-Cycloalkyl bedeuten, oder wobei R81 und R82 zusammen und unter Einschluß des Stickstoffatoms, an das beide gebunden sind, einen 1-Piperidyl- oder 1-Hexahydroazepinylrest darstellen,

sowie die Salze dieser Verbindungen.

Eine Ausgestaltung der bevorzugten Verbindungen der Formel I sind solche, in denen

R1 Methyl bedeutet,
R2 Ethoxy bedeutet,
R3 Methoxy oder Ethoxy bedeutet,

R4 einen durch R5 und R6 substituierten Phenylrest darstellt, wobei
R5 Wasserstoff bedeutet,
R6 CO-R7 oder CO-R8 bedeutet, wobei
R7 1-4C-Alkoxy bedeutet und
R8 N(R81)R82 bedeutet, wobei R81 und R82 unabhängig voneinander 1-4C-Alkyl oder 5-7C-Cycloalkyl bedeuten, oder wobei R81 und R82 zusammen und unter Einschluß des Stickstoffatoms, an das beide gebunden sind, einen 1-Piperidyl- oder 1-Hexahydroazepinylrest darstellen,

sowie die Salze dieser Verbindungen.

Besonders bevorzugte Verbindungen der Formel I sind solche, in denen

R1 Methyl bedeutet,
R2 Ethoxy oder Propoxy bedeutet,
R3 Methoxy bedeutet,
R4 einen durch R5 und R6 substituierten Phenylrest darstellt, wobei
R5 Wasserstoff bedeutet,
R6 CO-R8 bedeutet, wobei
R8 N(R81)R82 bedeutet, wobei R81 und R82 unabhängig voneinander 1-4C-Alkyl bedeuten,

sowie die Salze dieser Verbindungen.

Bei den Verbindungen der Formel I handelt es sich um chirale Verbindungen mit Chiralitätszentren in den Positionen 2, 4a und 10b

Numerierung:

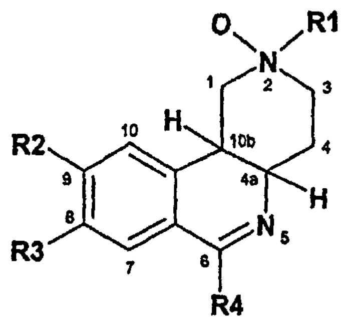

(I)

Gegenstand der Erfindung sind alle acht denkbaren Diastereomeren In jedem beliebigen Mischungsverhältnis zueinander. Bevorzugt sind die Verbindungen der Formel I, in denen die Wasserstoffatome in den Positionen 4a und 10b cis-ständig zueinander sind.

Besonders bevorzugt sind in diesem Zusammenhang solche Verbindungen der Formel I, die In den Positionen 4a und 10b dieselbe absolute Konfiguration haben wie die als Ausgangsprodukt einsetzbare Verbindung (-)-cis-4-Amino-3-(3-ethoxy-4-methoxyphenyl)-1-methylpiperidin-dihydrochlorid mit dem optischen Drehwert $[\alpha]_D^{20}$ = -65,5° (c=1, Methanol).

Weiterer Gegenstand der Erfindung ist ein Verfahren zur Herstellung der Verbindungen der Formel I, worin R1, R2, R3 und R4 die oben angegebenen Bedeutungen besitzen, und ihrer Salze. Das Verfahren ist dadurch gekennzeichnet, daß man Verbindungen der Formel II,

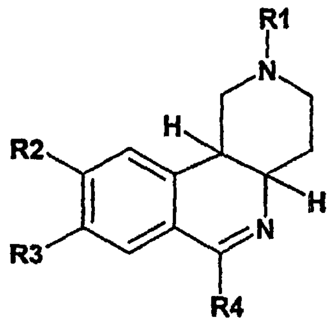

(II)

in denen R1. R2, R3 und R4 die oben angegebenen Bedeutungen besitzen, einer N-Oxidation unterwirft und gewünschtenfalls anschließend die erhaltenen Verbindungen der Formel I in ihre Salze überführt, oder daß man gewünschtenfalls anschließend erhaltene Salze der Verbindungen der Formel I in die freien Verbindungen überführt.

Die N-Oxidation erfolgt auf eine dem Fachmann vertraute Weise, z.B. mit Hilfe von Wasserstoffperoxid in Methanol oder mit Hilfe von m-Chlorperoxibenzoesäure in Dichlormethan bei Raumtemperatur. Welche Reaktionsbedingungen für die Durchführung des Verfahrens im einzelnen erforderlich sind, ist dem Fachmann aufgrund seines Fachwissens geläufig.

Gewünschtenfalls können erhaltene Verbindungen der Formel I durch Derivatisierung in weitere Verbindungen der Formel I übergeführt werden. Beispielsweise können aus Verbindungen der Formel I, worin R4 einen durch R5 und R6 substituierten Phenylrest und R6 eine Estergruppe darstellt durch saure oder alkalische Verseifung die entsprechenden Säuren erhalten werden oder durch Umsetzung mit Aminen der Formel HN(R81)R82, worin R81 und R82 die oben angegebenen Bedeutungen besitzen, die entsprechenden Amide dargestellt werden. Die Umsetzungen erfolgen zweckmäßigerweise analog dem Fachmann bekannter Methoden.

Verbindungen der Formel II, worin R1, R2, R3 und R4 die oben angegebenen Bedeutungen haben, können aus den entsprechenden Verbindungen der Formel III

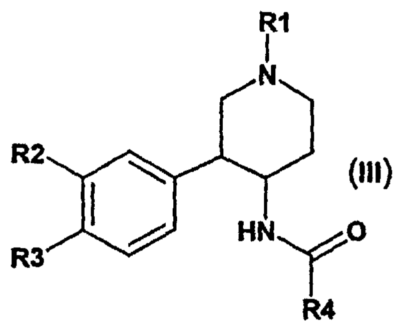

(III)

worin R1, R2, R3 und R4 die oben angegebenen Bedeutungen haben, durch eine Cyclokondensationsreaktion hergestellt werden.

Die Cyclokondensation erfolgt ebenfalls auf eine dem Fachmann an sich bekannte Weise gemäß Bischler-Napieralski (z.B. so, wie in J. Chem. Soc., **1956**, 4280-4282 beschrieben) in Gegenwart eines geeigneten Kondensationsmittels, wie beispielsweise Polyphosphorsäure, Phosphorpentachlorid, Phosphortrichtorid, Phosphorpentoxid, Thionylchlorid oder bevorzugt Phosphoroxytrichlorid, in einem geeigneten inerten Lösungsmittel, z.B. in einem chlorierten Kohlenwasserstoff wie Chloroform, oder in einem cyclischen Kohlenwasserstoff wie Toluol oder Xylol, oder einem sonstigen inerten Lösungsmittel wie Acetonitril, oder ohne weiteres Lösungsmittel unter Verwendung eines Überschusses an Kondensationsmittel, vorzugsweise bei erhöhter Temperatur, insbesondere bei der Siedetemperatur des verwendeten Lösungs- bzw. Kondensationsmittels,

Enantiomerenreine Verbindungen der Formel II können in bekannter Weise (beispielsweise durch Herstellung und Trennung entsprechender diastereoisomerer Verbindungen) separiert oder durch stereoselektive Synthesemethoden hergestellt werden. Solche Trennverfahren und Synthesemethoden sind beispielsweise in der EP 247 971 und in der DE 42 17 401 beschrieben.

Verbindungen der Formel III, worin R1, R2, R3 und R4 die oben angegebenen Bedeutungen haben, sind aus

den entsprechenden Verbindungen der Formel IV,

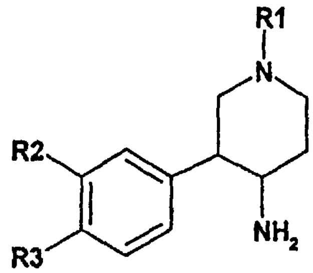

(IV)

worin R1, R2 und R3 die oben angegebenen Bedeutungen haben, durch Umsetzung mit Verbindungen der Formel R4-CO-X, worin R4 die oben angegebene Bedeutung hat und X eine geeignete Abgangsgruppe, vorzugsweise ein Chloratom darstellt, zugänglich. Beispielsweise wird die Benzoylierung wie in den nachfolgenden Beispielen nach dem Einhorn-Verfahren, der Schotten-Baumann-Variante oder wie in J. Chem. Soc. (C), **1971**, 1805-1808 beschrieben, durchgeführt.

Die Herstellung von cis/trans-Racemat-Gemischen und von reinen cis-Racematen von Verbindungen der Formel IV ist beispielsweise im USP 3,899,494, in DE-OS 21 23 328 und in DE-OS 16 95 782 beschrieben. Reine cis-Enantiomere der Verbindungen der Formel IV können beispielsweise nach den Verfahren erhalten werden, wie sie in EP 0 247 971 und in DE 42 17 401 offenbart sind.

Verbindungen der Formel R4-CO-X sind entweder bekannt oder können auf bekannte Weise hergestellt werden.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel (z. B. einem Keton, wie Aceton, Methylethylketon oder Methylisobutylketon, einem Ether, wie Diethylether, Tetrahydrofuran oder Dioxan, einem chlorierten Kohlenwasserstoff, wie Methylenchlorid oder Chloroform, oder einem niedermolekularen aliphatischen Alkohol wie Ethanol oder Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die nachfolgenden Beispiele dienen der näheren Erläuterung der Erfindung ohne sie einzuschränken. Ebenso können weitere Verbindungen der Formel 1, deren Herstellung nicht explizit beschrieben ist, in analoger oder in einer dem Fachmann an sich vertrauten Weise unter Anwendung üblicher Verfahrenstechniken hergestellt werden.

In den Beispielen steht Schmp. für Schmelzpunkt, h für Stunde(n), RT für Raumtemperatur, SF für Summenformel, MG für Molgewicht, Ber. für Berechnet, Gef. für Gefunden. Die in den Beispielen genannten Verbindungen und ihre Salze sind bevorzugter Gegenstand der Erfindung.

## Beispiele

### Endprodukte

#### 1. cis-9-Ethoxy-8-methoxy-2-methyl-6-(4-diisopropylaminocarbonylphenyl)-1,2,3,4,4a,10b-hexahydrobenzo[c][1,6]naphthyridin-N-2-oxid

Eine Lösung von 5,0 g (-)-cis-9-Ethoxy-8-methoxy-6-(4-diisopropylaminocarbonylphenyl)-2-methyl-1,2,3,4,4a,10b-hexahydrobenzo[c][1,6]naphthyridin in 40 ml Methanol wird mit 6 ml 30 % Wasserstoffperoxid ca. 2 Tage bei RT gerührt. Nach vollständiger Oxidation (DC-Kontrolle) versetzt man das Reaktionsgemisch mit 7 g festem Natriumsulfit und rührt noch ca. 1 h bei RT nach. Nach dem Absaugen des Reaktionsgemisches extrahiert man das Filtrat mit Dichlormethan, wäscht die organische Phase mit gesättigter Natriumhydrogencarbonatlösung, anschließend

mit Wasser und trocknet sie über Natriumsulfat. Nach dem Absaugen und Einengen der Produktlösung kristallisiert man den erhaltenen festen Rückstand in einem Aceton/Ethylacetat-Gemisch (10+1) um. Man erhält 2,7 g der Titelverbindung als farblose feine Kristalle vom Schmp. 195-198°C.
SF: $C_{29}$ $H_{39}$ $N_3$ $O_4$ x 1,38 $H_2O$; MG: 518,52

| | |
|---|---|
| Elementaranalyse | Ber.: C 67,19 H 8,12 N 8,11 |
| | Gef.: C 67,18 H 8,00 N 8,13 |

Ausgehend von den entsprechenden Ausgangsverbindungen werden die folgenden zwei Endprodukte analog zu Beispiel 1 hergestellt:

**2. cis-9-Ethoxy-2-methyl-8-methoxy-6-(4-dibutylaminocarbonylphenyl)-1,2,3,4,4a,10b-hexahydrobenzo[c][1,6]naphthyridin-N-2-Oxid**

SF: $C_{31}$ $H_{43}$ $N_3$ $O_4$; MW: 521,7; Schmp. 66 - 80 °C (langsames Zerfließen), über 80 °C Zersetzung

**3. cis-8-Methoxy-2-methyl-9-propoxy-6-(4-dibutylaminocarbonylphenyl)-1,2,3,4,4a,10b-hexahydrobenzo[c][1,6]naphthyridin-N-2-Oxid**

SF: $C_{32}$ $N_{45}$ $N_3$ $O_4$; MW: 535,7; Schmp. 104 - 108 °C (langsames Zerfließen), über 108 °C Zersetzung

**Ausgangsverbindungen**

**A1. (-)-cis-9-Ethoxy-8-methoxy-6-(4-diisopropylaminocarbonylphenyl)-2-methyl-1,2,3,4,4a,10b-hexahydrobenzo[c][1,6]naphthyridin-hydrochlorid**

6,36 g (-)-cis-Terephthalsäure-N,N-diisopropyl-N'-[3-(3-ethoxy-4-methoxyphenyl)-1-methylpiperidin-4-yl]-diamid werden in 100 ml Acetonitril und 12 ml Phosphoroxytrichlorid 15 h unter Rückfluß zum Sieden erhitzt. Nach Abdestillieren des überschüssigen Phosphoroxytrichlorids verteilt man den Rückstand zwischen Dichlormethan und gesättigter Natriumhydrogencarbonatlösung. Die organische Phase wird mit Wasser gewaschen, über Natriumsulfat getrocknet und eingeengt. Den festen Rückstand reinigt man durch Kieselgelchromatographie, separiert die Hauptproduktfraktion und engt diese ein. Den festen Rückstand löst man in wenig Methanol, versetzt die Lösung mit 1 Äquivalent wäßriger HCl und engt ein. Der feste Rückstand wird in Methanol/Diethylether umkristallisiert. Man erhält 4,51 g der Titelverbindung als Hydrochlorid-0,6-Hydrat mit dem Schmp. 175-179°C (unscharf).
SF: $C_{29}$ $H_{39}$ $N_3$ $O_3$ x HCl x 0,6 $H_2O$; MG: 524,92

| | |
|---|---|
| Elementaranalyse | Ber.: C 66,36 H 7,91 Cl 6,75 N 8,01 |
| | Gef.: C 66,28 H 7,99 Cl 6,87 N 7,97 |
| Optischer Drehwert | $[\alpha]_D^{20}$ = -42,7° (c=1, Methanol). |

Die freie Base wird aus dem Hydrochlorid durch Extraktion mit Dichlormethan nach Behandeln mit verdünnter Natronlauge erhalten.
Ausgehend von den entsprechenden Ausgangsverbindungen werden A2 und A3 analog zu Beispiel A1 hergestellt:

**A2. (-)-cis-9-Ethoxy-8-methoxy-2-methyl-6-(4-dibutylaminocarbonylphenyl)-1,2,3,4,4a,10b-hexahydrobenzo[c][1,6]naphthyridin-hydrochlorid**

SF: $C_{31}$ $H_{43}$ $N_3$ $O_3$ x 1,1 HCl x 1,17 $H_2O$; MG: 566,82; Schmp.: 104-112°C (fest aufgeschäumtes Produkt, langsames Zerfließen);

| | |
|---|---|
| Elementaranalyse | Ber.: C 65,68 H 8,26 Cl 6,88 N 7,41 |
| | Gef.: C 65,80 H 8,09 Cl 6,97 N 7,49 |
| Optischer Drehwert | $[\alpha]_D^{20}$ = -16.2° (c = 1, Methanol). |

**A3. (-)-cis-8-Methoxy-2-methyl-9-propoxy-6-(4-dibutylaminocarbonylphenyl)-1,2,3,4,4a,10b-hexahydrobenzo[c][1,6]naphthyridin-hydrochlorid**

SF: $C_{32} H_{45} N_3 O_3$ x 1,05 HCl x 0,58 $H_2O$; MW: 568,5; Schmp. 105-112 °C (unscharf);

| | |
|---|---|
| Elementaranalyse | Ber.: C 67,64 H 8,37 Cl 6,55 N 7,39 |
| | Gef.: C 67,55 H 8,29 Cl 6,68 N 7,31 |
| Optischer Drehwert | $[\alpha]_D^{20}$ = -15,8° (c=1, Methanol). |

**B1. (-)-cis-Terephthalsäure-N,N-diisopropyl-N'-[3-(3-ethoxy-4-methoxyphenyl)-1-methylpiperidin-4-yl]-diamid**

Zur Lösung von 1,5 g (-)-cis-4-Amino-3-(3-ethoxy-4-methoxyphenyl)-1-methylpiperidin (hergestellt durch Extraktion der freien Base mit Dichlormethan nach dem Behandeln des entsprechenden Dihydrochlorids ($[\alpha]_D^{20}$ = -65,5°, c=1, Methanol) mit verdünnter Natronlauge] in 60 ml Dichlormethan und 0,9 ml Triethylamin wird bei RT eine Lösung von 4-Diisopropylaminocarbonylbenzoylchlorid (hergestellt aus 1,5 g 4-Diisopropylaminocarbonylbenzoesäure und Thionylchlorid) in 60 ml Dichlormethan innerhalb von 10 Min. zugetropft. Nach ca. 2 h Rühren extrahiert man mit ca. 50 ml gesättigter Natriumhydrogencarbonatlösung, wäscht die organische Phase noch zweimal mit je 50 ml Wasser und trocknet sie über Natriumsulfat. Der nach dem Einengen verbleibende zäh-viskose Rückstand wird durch Säulenchromatographie gereinigt. Die im Vakuum eingeengte Hauptproduktfraktion ergibt einen fest aufschäumenden Rückstand, der in einem Gemisch aus Methanol und Diethylether (ca. 1 + 1 Vol.) umkristallisiert wird. Man erhält 2,7 g der Titelverbindung mit dem Schmp. 75-82°C (unscharfer Bereich, erstarrter Schaum).
SF: $C_{29} H_{41} N_3 O_4$; MG: 495,67

| | |
|---|---|
| Optischer Drehwert | $[\alpha]_D^{20}$ = -60,1 ° (c=1, Methanol). |

Die nachfolgende Verbindung wird analog dem in DE 42 17 401 beschriebenen Verfahren erhalten, wenn in den dort beschriebenen Beispielen anstatt der 3,4-Dimethoxy- die 3-Ethoxy-4-methoxy-Verbindungen eingesetzt werden:

**C1. (-)-cis-4-Amino-3-(3-ethoxy-4-methoxyphenyl)-1-methylpiperidin-dihydrochlorid**

SF: $C_{15}H_{24} N_2O_2$ x 2 HCl x 0,96 $H_2O$; MG: 354,52; Schmp.: 252-254°C;

| | |
|---|---|
| Optischer Drehwert | $[\alpha]_D^{20}$ = -65,5° (c=1, Methanol). |

## Gewerbliche Anwendbarkeit

Die erfindungsgemäßen Verbindungen besitzen wertvolle pharmakologische Eigenschaften, die sie gewerblich verwertbar machen. Als selektive Inhibitoren des Typs 3 und 4 der Zyklisch-Nukleotid-Phosphodiesterase (PDE3, PDE4) eignen sie sich einerseits als Bronchialtherapeutika (zur Behandlung von Atemwegsobstruktionen aufgrund ihrer dilatierenden und zilienstimulierenden aber auch aufgrund ihrer atemfrequenz- bzw. atemantriebssteigernden Wirkung) andererseits jedoch vor allem zur Behandlung von Erkrankungen entzündlicher Natur, z.B. der Atemwege (Asthma-Prophylaxe), der Haut, des Darms, der Augen und der Gelenke, die vermittelt werden durch Mediatoren wie Interferone, Mitglieder der Tumor Nekrose-Faktorenfamilie, Interleukine, Chemokine, Kolonie-stimulierende Faktoren, Wachstumsfaktoren, Lipidmediatoren (z.B. u.a. PAF, plättchenaktivierender Faktor), bakterielle Faktoren (z.B. LPS), Immunglobuline, Sauerstoffradikale und Verwandte (z.B. Stickstoffmonoxid NO), biogene Amine (z.B. Histamin, Serotonin), Kinine (z.B. Bradykinin), neurogene Mediatoren (wie Substanz P, Neurokinin), Proteine wie z.B. Granulainhaltsstoffe von Leukozyten (u.a. kationische Proteine von Eosinophilen) und Adhärenzproteine (z.B. Integrine). Die erfindungsgemäßen Verbindungen besitzen glattmuskelzell-relaxierende Wirkung, z.B. im Bereich des Bronchialsystems, des Blutkreislaufs, und der ableitenden Harnwege. Desweiteren besitzen sie zilienfrequenz-steigernde Wirkung, z.B. im Bronchialsystem.
Hierbei zeichnen sich die erfindungsgemäßen Verbindungen durch eine geringe Toxizität, eine gute Humanakzeptanz, eine gute enterale Resorption und eine hohe Bioverfügbarkeit, eine große therapeutische Breite, das Fehlen wesentlicher Nebenwirkungen und eine gute Wasserlöslichkeit aus.
Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale, Emphysema, COPD); Erkrankungen mit einer Einschränkung der Zilientätigkeit oder verstärkten Anforderungen an die ziliäre Clearance (Bronchitis, Mucoviscidose), Dermatosen (vor allem proliferativer, entzündlicher und allergischer Art) wie beispielsweise Psoriasis (vuigaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), systemischer Lupus erythematosus, Erkrankungen des Immunsystems (AIDS), einschließlich AIDS-bedingter Enzephalopathien, Autoimmun-Erkrankungen wie Diabetes mellitus (Typ I, Autoimmundiabetes), Multiple Sklerose und der Formenkreis Virus-, Bakterien- oder Parasiten-induzierter Demyelinisierungs-Krankheiten, zerebrale Malaria oder Lymes Erkrankung, Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; ferner Erkrankungen des Zentralnerversystems wie Gedächtnisstörungen und Alzheimer's Erkrankung, Candidiasis, Leishmaniosen und Lepra.

Aufgrund ihrer gefäßrelaxierenden Wirksamkeit können die erfindungsgemäßen Verbindungen auch zur Behandlung von Bluthochdruckerkrankungen verschiedener Genese wie z.B. pulmonarer Hochdruck und den damit zusammenhängenden Begleiterscheinungen, zur Behandlung erektiler Dysfunktion oder Koliken der Nieren und der Harnleiter im Zusammenhang mit Nierensteinen, verwendet werden.

Aufgrund ihrer cAMP-steigernden Wirkung können sie aber auch für Erkrankungen des Herzens, die durch PDE-Hemmstoffe behandelt werden können, wie beispielsweise Herzinsuffizienz, sowie als antithrombotische, die Plättchen-Aggregation hemmende Substanzen verwendet werden.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe von Krankheiten, insbesondere den genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Ein weiterer Gegenstand der Erfindung ist ein Handelsprodukt, bestehend aus einem üblichen Sekundärpackmittel, einem das Arzneimittel enthaltenden Primärpackmittel (beispielsweise eine Ampulle oder ein Blister) und gewünschtenfalls einen Beipackzettel, wobei das Arzneimittel antagonistische Wirkung gegen zyklisch-nukleotid Phosphodiesterasen des Typs 3 und 4 zeigt und zur Abschwächung der Symptome von Krankheiten führt, die in Zusammenhang mit zyklisch-nukleotid Phoshodiesterasen des Typs 3 und 4 stehen, und wobei auf dem Sekundärpackmittel und/oder auf dem Beipackzettel des Handelsprodukts auf die Eignung des Arzneimittels zur Prophylaxe oder Behandlung von Krankheiten, die im Zusammenhang mit zyklisch-nukleotid Phosphodiesterasen des Typs 3 und 4 stehen, hingewiesen wird, und wobei das Arzneimittel ein oder mehrere erfindungsgemäße Verbindungen der Formel I enthält. Das Sekundärpackmittel, das das Arzneimittel enthaltende Primärpackmittel und der Beipackzettel entsprechen ansonsten dem, was der Fachmann als Standard für Arzneimittel dieser Art ansehen würde.

In vorteilhafter Weise eignen sich die erfindungsgemäßen Substanzen auch zur Kombination mit anderen Substanzen, die eine Stimulation von cAMP bewirken, wie Prostaglandine (PGE2, PGI2 bzw. Prostazyklin) und ihre Abkömmlinge, direkte Adenylatzyktase-Stimulatoren wie Forskolin und verwandte Substanzen, bzw. mittelbar die Adenylatzyklase stimulierende Substanzen wie Katecholamine und adrenerge Rezeptoragonisten, insbesondere Beta-Mimetika. Sie entfalten dabei in Kombination auf Grund ihrer den cAMP-Abbau hemmenden Wirkung eine synergistische, überadditive Wirksamkeit. Diese kommt z.B. bei ihrer Anwendung in Kombination mit PGE2 zur Behandlung der pulmonalen Hypertonie zum Tragen.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise

zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,1 und 3 mg pro Tag. Die übliche Dosis bei systemischer Therapie (p.o. oder i.v.) liegt zwischen 0,01 und 10 mg pro Kilogramm und Tag.

Biologische Untersuchungen

Bei der Untersuchung der PDE4-Hemmung auf zellulärer Ebene kommt der Aktivierung von Entzündungszellen besondere Bedeutung zu. Als Beispiel sei die FMLP (N-formyl-methionylleucyl-phenylalanin)-induzierte Superoxid-Produktion von neutrophilen Granulozyten genannt, die als Luminol-verstärkte Chemilumineszenz gemessen werden kann. [Mc Phail LC, Strum SL, Leone PA und Sozzanl S, The neutrophil respiratory burst mechanism. In "Immunology Series" **1992,** 57, 47-76; ed. Coffey RG (Marcel Decker, Inc., New York-Basel-Hong Kong)].

Substanzen, welche die Chemilumineszenz sowie die Zytokinsekretion und die Sekretion entzündungssteigernder Mediatoren an Entzündungszellen, insbesonders neutrophilen und eosinophilen Granulozyten, T-Lymphozyten, Monozyten und Makrophagen hemmen, sind solche, welche die PDE4 oder PDE3 und PDE4 hemmen. Das letztgenannte Isoenzym der Phosphodiesterase-Familien Ist besonders in Granulozyten vertreten. Dessen Hemmung führt zur Erhöhung der intrazellulären zyklischen AMP-Konzentration und damit zur Hemmung der zellulären Aktivierung. Die PDE4-Hemmung durch die erfindungsgemäßen Substanzen ist damit ein zentraler Indikator für die Unterdrückung von entzündlichen Prozessen. **(Giembycz MA,** Could isoenzyme-selective phosphodiesterase inhibitors render bronchodilatory therapy redundant in the treatment of bronchial asthma?; Biochern Pharmacol **1992,** 43, 2041-2051; **Torphy TJ** et al., Phosphodiesterase Inhibitors: new opportunities for treatment of asthma. Thorax **1991**, 46, 512-523; **Schudt C** et al., Zardaverine: a cyclic AMP PDE3/4 inhibitor. In "New Drugs for Asthma Therapy", 379-402, Birkhäuser Verlag Basel 1991; **Schudt C** et al., Influence of selective phosphodiesterase inhibitors on human neutrophil functions and levels of cAMP and Ca; Naunyn-Schmledebergs Arch Pharmacol **1991,** 344, 682-690; **Tenor H** and **Schudt** C, Analysis of PDE isoenzyme profiles in cells and tissues by pharmacological methods. In "Phosphodiesterase Inhibitors", 21-40, "The Handbook of Immunopharmacology", Academic Press 1996. **Hatzelmann** A et al., **Enzymatic** and functional aspects of dual-selective PDE3/4-inhibitors. In "Phosphodiesterase Inhibitors", 147-160, "The Handbook of Immunopharmacology", Academic Press, 1996.

**A. Methodik**

1. Hemmung der PDE Isoenzyme

Die PDE-Aktivität wurde gemäß Thompson et al. (1) mit einigen Modifikationen (2) bestimmt. Die Prüfproben enthielten 40 mM Tris-HCl (pH 7,4), 5 mM $MgCl_2$, 0,5 μM cAMP oder cGMP, [$^3$H]cAMP oder [$^3$H]cGMP (ca. 50.000 cpm/Probe), die unten näher beschriebenen PDE isoenzym-spezifischen Zusätze, die angegebenen Konzentrationen an Hemmstoff und ein Aliquot der Enzymlösung bei einem Gesamtprobenvolumen von 200 μl. Stammlösungen der zu untersuchenden Verbindungen in DMSO wurden in solchen Konzentrationen hergestellt, daß der DMSO-Gehalt - zur Vermeidung einer Beeinflussung der PDE-Aktivität - in den Prüfproben 1 Vol-% nicht überschritt. Nach 5-minütiger Vorinkubation bei 37°C wurde die Reaktion durch Zugabe des Substrates (cAMP oder cGMP) in Gang gesetzt. Die Proben wurden für weitere 15 Min. bei 37°C inkubiert. Durch Zugabe von 50 μl 0,2 N HCl wurde die Reaktion abgebrochen. Nach 10-minütiger Kühlung auf Eis und Zugabe von 25 μg 5'-Nukleotidase (Schlangengift von Crotalus atrox)

wurde erneut für 10 Min. bei 37°C inkubiert und die Proben dann auf QAE Sephadex A-25-Säulen aufgetragen. Die Säulen wurden mit 2 ml 30 mM Ammoniumformiat (pH 6,0) eluiert. Die Radioaktivität des Eluats wurde gemessen und um die entsprechenden Leerwerte korrigiert. Der Anteil an hydrolysiertem Nucleotid überschritt in keinem Fall 20 % der ursprünglichen Substratkonzentration.

PDE1 ($Ca^{2+}$/Calmodulin-abhängig) aus Rinderhirn: Die Hemmung dieses isoenzyms wurde in Gegenwart von $Ca^{2+}$ (1 mM) und Calmodulin (100 nM) unter Verwendung von cGMP als Substrat untersucht (3).

PDE2 (cGMP-stimuliert) aus Rattenherzen wurde chromatografisch gereinigt [Schudt et al. (4)] und in Gegenwart von cGMP (5 μM) unter Verwendung von cAMP als Substrat untersucht.

PDE3 (cGMP-inhibiert) und PDE5 (cGMP-spezifisch) wurden in Homogenaten von menschlichen Blutplättchen [Schudt et al. (4)] unter Verwendung von cAMP bzw. cGMP als Substrat untersucht.

PDE4 (cAMP-spezifisch) wurde im Zytosol von humanen polymorphonuclearen Leukozyten (PMNL) [isoliert aus Leukozytenkonzentraten, siehe Schudt et al. (5)] unter Verwendung von cAMP als Substrat untersucht. Der PDE3-Hemmstoff Motapizon (1 μM) wurde verwendet um die von verunreinigenden Blutplättchen ausgehende PDE3 Aktivität zu unterdrücken.

2. Statistik

Die $IC_{50}$-Werte wurden aus den Konzentrations-Hemmkurven durch nichtlineare Regression unter Verwendung des Programms GraphPad InPlot™ (GraphPad Software Inc., Philadelphia, USA) ermittelt.

3. Literatur

(1) Thompson W.J., Terasaki W. L., Epstein P. M. and Strada S. J., Assay of cyclic nucleotide phosphodiesterase and resolution of multiple molecular forms of the enzyme; Adv. Cycl. Nucl. Res. **1979,** 10, 69-92

(2) Bauer A.C. und Schwabe U., An improved assay of cyclic 3',5'-nucleotide phosphodiesterase with QAE Sephadex A-25; Naunyn-Schmiedeberg's Arch. Pharmacol. **1980**, 311, 193-198

(3) Gietzen K., Sadorf 1. und Bader H., A model for the regulation of the calmodulin-dependent enzymes erythrocyte $Ca^{2+}$-transport ATPase and brain phosphodiesterase by activators and Inhibiors; Biochem. J. **1982**, 207, 541-548

(4) Schudt C., Winder S., Müller B. und Ukena D., Zardaverine as a selective inhibitor of phosphodiesterase isoenzymes; Blochem. Pharmacol. **1999,** 42, 153-162

(5) Schudt C., Winder S., Forderkunz S., Hatzelmann A. und Ullrich V., Influence of selective phosphodiesterase inhibitors on human neutrophil functions and levels of cAMP and Ca; Naunyn-Schmiedeberg's Arch. Pharmacol. **1991**, 344, 682-690

**B. Ergebnisse**

In der nachfolgenden Tabelle 1 sind die gemäß Punkt A1 ermittelten Hemmkonzentrationen [Hemmkonzentrationen als -log $IC_{50}$ (mol/l)] für einige erfindungsgemäße Verbindungen angegeben. Die Nummern der Verbindungen entsprechen den Nummern der Beispiele.

**Tabelle 1**

| Verbindung | PDE4 | PDE3 |
|---|---|---|
| | [-log $IC_{50}$, mol/l] | |
| 1 | 7,20 | 6,11 |
| 2 | 7,98 | 5,97 |
| 3 | 7,53 | 5,68 |

**Patentansprüche**

1. Verbindungen der Formel I,

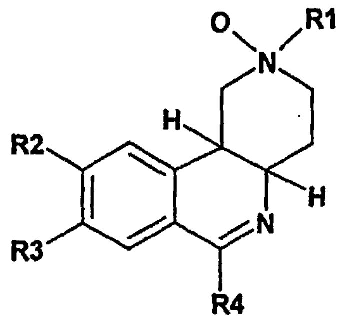

(I)

worin

R1 1-40-Alkyl bedeutet,
R2 Hydroxy, 1-4C-Alkoxy, 3-7C-Cycloalkoxy, 3-7C-Cycloalkylmethoxy oder ganz oder überwiegend durch Fluor substituiertes 1-4C-Alkoxy bedeutet,
R3 Hydroxy, 1-4C-Alkoxy, 3-7C-Cycloalkoxy, 3-7C-Cycloalkylmethoxy oder ganz oder überwiegend durch Fluor substituiertes 1-40-Alkoxy bedeutet,

oder worin

R2 und R3 gemeinsam eine 1-2C-Alkylendioxygruppe bedeuten,
R4 einen durch R5 und R6 substituierten Phenylrest darstellt, wobei
R5 Wasserstoff, Hydroxy, Halogen, Nitro, 1-4C-Alkyl, Trifluormethyl oder 1-4C-Alkoxy bedeutet,
R6 CO-R7 oder CO-R8 bedeutet, wobei
R7 Hydroxy, 1-8C-Alkoxy, 3-7C-Cycloalkoxy oder 3-7C-Cycloalkylmethoxy bedeutet und
R8 N(R81)R82 bedeutet, wobei R81 und R82 unabhängig voneinander Wasserstoff, 1-7C-Alkyl, 3-7C-Cycloalkyl oder 3-7C-Cycloalkylmethyl bedeuten, oder wobei R81 und R82 zusammen und unter Einschluß des Stickstoffatoms, an das beide gebunden sind, einen 1-Pyrrolidinyl-, 1-Piperidyl-, 1-Hexahydroazepinyl- oder 4-Morpholinylrest darstellen,

sowie die Salze dieser Verbindungen.

2. Verbindungen der Formel I nach Anspruch 1, in denen

R1 Methyl bedeutet,
R2 1-40-Alkoxy bedeutet,
R3 1-40-Alkoxy bedeutet,
R4 einen durch R5 und R6 substituierten Phenylrest darstellt, wobei
R5 Wasserstoff, Hydroxy, 1-40-Alkyl oder 1-4C-Alkoxy bedeutet,
R6 CO-R7 oder CO-R8 bedeutet, wobei

R7 Hydroxy, 1-8C-Alkoxy oder 3-7C-Cycloalkoxy bedeutet und
R8 N(R81)R82 bedeutet, wobei R81 und R82 unabhängig voneinander Wasserstoff, 1-7C-Alkyl oder 3-7C-Cycloalkyl bedeuten, oder wobei R81 und R82 zusammen und unter Einschluß des Stickstoffatoms, an das beide gebunden sind, einen 1-Piperidyl-, 1-Hexahydroazepinyl- oder 4-Morpholinylrest darstellen,

sowie die Salze dieser Verbindungen.

3. Verbindungen der Formel I nach Anspruch 1, in denen

R1 Methyl bedeutet,
R2 Methoxy, Ethoxy oder Propoxy bedeutet,
R3 Methoxy oder Ethoxy bedeutet,
R4 einen durch R5 und R6 substitulerten Phenylrest darstellt, wobei
R5 Wasserstoff bedeutet,
R6 CO-R7 oder CO-R8 bedeutet, wobei
R7 Hydroxy oder 1-8C-Alkoxy bedeutet und
R8 N(R81)R82 bedeutet, wobei R81 und R82 unabhängig voneinander Wasserstoff oder 1-40-Alkyl oder 5-7C-Cycloalkyl bedeuten, oder wobei R81 und R82 zusammen und unter Einschluß des Stickstoffatoms, an das beide gebunden sind, einen 1-Piperidyl-, 1-Hexahydroazepinyl- oder 4-Morpholinylrest darstellen,

sowie die Salze dieser Verbindungen.

**4.** Verbindungen der Formel I nach Anspruch 1, in denen

R1 Methyl bedeutet,
R2 Ethoxy bedeutet,
R3 Methoxy oder Ethoxy bedeutet,
R4 einen durch R5 und R6 substitulerten Phenylrest darstellt, wobei
R5 Wasserstoff bedeutet,
R6 CO-R7 oder CO-R8 bedeutet, wobei
R7 1-4C-Alkoxy bedeutet und
R8 N(R81)R82 bedeutet, wobei R81 und R82 unabhängig voneinander 1-40-Alkyl oder 5-7C-Cycloalkyl bedeuten, oder wobei R81 und R82 zusammen und unter Einschluß des Stickstoffatoms, an das beide gebunden sind, einen 1-Piperidyl- oder 1-Hexahydroazepinylrest darstellen,

sowie die Salze dieser Verbindungen.

**5.** Verbindungen der Formel I nach Anspruch 1, in denen

R1 Methyl bedeutet,
R2 Ethoxy oder Propoxy bedeutet,
R3 Methoxy bedeutet,
R4 einen durch R5 und R6 substituierten Phenylrest darstellt, wobei
R5 Wasserstoff bedeutet,
R6 CO-R8 bedeutet, wobei
R8 N(R81)R82 bedeutet, wobei R81 und R82 unabhängig voneinander 1-4C-Alkyl bedeuten,

sowie die Salze dieser Verbindungen.

**6.** Verbindungen der Formel I nach Anspruch 1, in denen die Wasserstoffatome in den Positionen 4a und 10b cis-ständig zueinander sind, sowie die Salze dieser Verbindungen.

**7.** Verbindungen der Formel I nach Anspruch 1, die in den Positionen 4a und 10b dieselbe absolute Konfiguration haben, wie die als Ausgangsprodukt einsetzbare Verbindung (-)-cis-4-Amino-3-(3-ethoxy-4-methoxyphenyl)-1-methylpiperidin-dihydrochlorid mit dem optischen Drehwert $[\alpha]_D^{20}$ = -65,5° (c=1, Methanol).

**8.** Verbindungen nach Anspruch 1 zur Anwendung bei der Behandlung von Krankheiten.

**9.** Arzneimittel enthaltend eine oder mehrere Verbindungen nach Anspruch 1 zusammen mit den üblichen pharmazeutischen Hilfs- und/oder Trägerstoffen.

**10.** Verwendung von Verbindungen nach Anspruch 1 zur Herstellung von Arzneimitteln für die Behandlung von Atemwegserkrankungen und/oder Dermatosen.

**Claims**

1. Compounds of the formula I

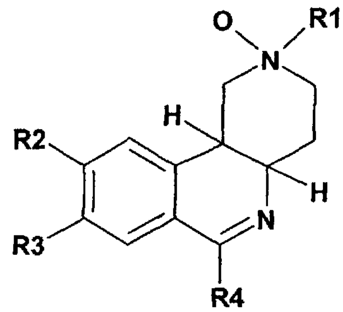

(I)

in which

R1 is 1-4C-alkyl,
R2 is hydroxyl, 1-4C-alkoxy, 3-7C-cycloalkoxy, 3-7C-cycloalkylmethoxy or 1-4C-alkoxy which is completely or predominantly substituted by fluorine,
R3 is hydroxyl, 1-4C-alkoxy, 3-7C-cycloalkoxy, 3-7C-cycloalkylmethoxy or 1-4C-alkoxy which is completely or predominantly substituted by fluorine,

or in which

R2 and R3 together are a 1-2C-alkylenedioxy group,
R4 is a phenyl radical which is substituted by R5 and R6, where
R5 is hydrogen, hydroxyl, halogen, nitro, 1-4C-alkyl, trifluoromethyl or 1-4C-alkoxy,
R6 is CO-R7 or CO-R8, where
R7 is hydroxyl, 1-8C-alkoxy, 3-7C-cycloalkoxy or 3-7C-cycloalkylmethoxy and
R8 is N(R81)R82, where R81 and R82 independently of one another are hydrogen, 1-7C-alkyl, 3-7C-cycloalkyl or 3-70-cycloalkylmethyl, or where R81 and R82, together and including the nitrogen atom to which both are bonded, are a 1-pyrrolidinyl, 1-piperidyl, 1-hexahydroazepinyl or 4-morpholinyl radical,

and to the salts of these compounds.

2. Compounds of the formula I according to claim 1 in which

R1 is methyl,
R2 is 1-4C alkoxy,
R3 is 1-4C-alkoxy,
R4 is a phenyl radical which is substituted by R5 and R6, where
R5 is hydrogen, hydroxyl, 1-4C-alkyl or 1-4C-alkoxy,
R6 is CO-R7 or CO-R8, where
R7 hydroxyl, 1-8C-alkoxy or 3-7C-cycloalkoxy and
R8 is N(R81)R82, where R81 and R82 independently of one another are hydrogen, 1-70-alkyl or 3-7C-cycloalkyl, or where R81 and R82, together and including the nitrogen atom to which both are bonded, are a 1-piperidyl, 1-hexahydroazepinyl or 4-morpholinyl radical,

and the salts of these compounds.

3. Compounds of the formula I according to claim 1 in which

R1 is methyl,
R2 is methoxy, ethoxy or propoxy,

R3 is methoxy or ethoxy,
R4 is a phenyl radical which is substituted by R5 and R6, where
R5 is hydrogen,
R6 is CO-R7 or CO-R8, where
R7 hydroxyl or 1-8C-alkoxy and
R8 is N(R81)R82, where R81 and R82 independently of one another are hydrogen or 1-4C-alkyl or 5-7C-cycloalkyl, or where R81 and R82, together and including the nitrogen atom to which both are bonded, are a 1-piperidyl, 1-hexahydroazepinyl or 4-morpholinyl radical,

and the salts of these compounds.

**4.** Compounds of the formula I according to claim 1 in which

R1 is methyl,
R2 is ethoxy,
R3 is methoxy or ethoxy,
R4 is a phenyl radical which is substituted by R5 and R6, where
R5 is hydrogen,
R6 is CO-R7 or CO-R8, where
R7 is 1-4C-alkoxy and
R8 is N(R81)R82, where R81 and R82 independently of one another are 1-4C-alkyl or 5-7C-cycloalkyl, or where R81 and R82, together and including the nitrogen atom to which both are bonded, are a 1-piperidyl or 1-hexahydroazepinyl radical,

and the salts of these compounds.

**5.** Compounds of the formula I according to claim 1 in which

R1 is methyl,
R2 is ethoxy or propoxy,
R3 is methoxy,
R4 is a phenyl radical which is substituted by R5 and R6, where
R5 is hydrogen,
R6 is CO-R8, where
R8 is N(R81)R82, where R81 and R82 independently of one another are 1-4C-alkyl,

and the salts of these compounds.

**6.** Compounds of the formula I according to claim 1 in which the hydrogen atoms in positions 4a and 10b are in the cis position relative to one another.

**7.** Compounds of the formula I according to claim 1 which in positions 4a and 10b have the same absolute configuration as the compound (-)-cis-4-amino-3-(3-ethoxy-4-methoxyphenyl)-1-methylpiperidine dihydrochloride having the optical rotation $[\alpha]_D^{20}$ = -65.5° (c=1, methanol) which can be employed as starting material.

**8.** Compounds according to claim 1 for the treatment of illnesses.

**9.** Medicaments containing one or more compounds according to claim 1 together with the usual pharmaceutical auxiliaries and/or carrier materials.

**10.** Use of compounds according to claim 1 for the production of medicaments for the treatment of airway disorders and/or dermatoses.

## Revendications

**1.** Composés de formule I

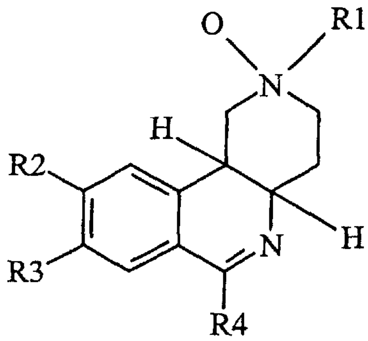

(I)

dans laquelle

R1 représente un groupe alkyle en $C_1$-$C_4$,
R2 représente un groupe hydroxy, alcoxy en $C_1$-$C_4$, cycloalcoxy en $C_3$-$C_7$, (cycloalkyle en $C_3$-$C_7$)méthoxy, ou un groupe alcoxy en $C_1$-$C_4$ dont la totalité ou la plupart des atomes d'hydrogène ont été remplacés par des atomes de fluor,
R3 représente un groupe hydroxy, alcoxy en $C_1$-$C_4$, cycloalcoxy en $C_3$-$C_7$, (cycloalkyle en $C_3$-$C_7$)méthoxy, ou un groupe alcoxy en $C_1$-$C_4$ dont la totalité ou la plupart des atomes d'hydrogène ont été remplacés par des atomes de fluor,

ou bien R2 et R3 représentent ensemble un groupe alkylènedioxy en $C_1$-$C_2$, et

R4 représente un groupe phényle portant des substituants R5 et R6, où
R5 représente un atome d'hydrogène ou d'halogène ou un groupe hydroxy, nitro, alkyle en $C_1$- $C_4$, trifluorométhyle ou alcoxy en $C_1$-$C_4$, et
R6 représente un groupe de formule -CO-R7 ou -CO-R8, où
R7 représente un groupe hydroxy, alcoxy en $C_1$-$C_8$, cycloalcoxy en $C_3$-$C_7$ ou (cycloalkyle en $C_3$- $C_7$)méthoxy, et
R8 représente un groupe de formule -N(R81)R82 où R81 et R82 représentent chacun, indépendamment l'un de l'autre, un atome d'hydrogène ou un groupe alkyle en $C_1$-$C_7$, cycloalkyle en $C_3$-$C_7$ ou (cycloalkyle en $C_3$-$C_7$)méthyle, ou bien R81 et R82 représentent ensemble, conjointement avec l'atome d'azote auquel ils sont tous les deux liés, un groupe 1-pyrrolidinyle, 1-pipéridyle, 1-hexahydroazépinyle ou 4-morpholinyle,

ainsi que les sels de tels composés.

**2.** Composés de formule I, conformes à la revendication 1, dans lesquels

R1 représente un groupe méthyle,
R2 représente un groupe alcoxy en $C_1$-$C_4$,
R3 représente un groupe alcoxy en $C_1$-$C_4$, et
R4 représente un groupe phényle portant des substituants R5 et R6, où
R5 représente un atome d'hydrogène ou un groupe hydroxy, alkyle en $C_1$-$C_4$ ou alcoxy en $C_1$-$C_4$, et
R6 représente un groupe de formule -CO-R7 ou -CO-R8, où
R7 représente un groupe hydroxy, alcoxy en $C_1$-$C_8$ ou cycloalcoxy en $C_3$-$C_7$, et
R8 représente un groupe de formule -N(R81 )R82 où R81 et R82 représentent chacun, indépendamment l'un de l'autre, un atome d'hydrogène ou un groupe alkyle en $C_1$-$C_7$ ou cycloalkyle en $C_3$-$C_7$, ou bien R81 et R82 représentent ensemble, conjointement avec l'atome d'azote auquel ils sont tous les deux liés, un groupe 1-pipéridyle, 1-hexahydroazépinyle ou 4-morpholinyle,

ainsi que les sels de ces composés.

**3.** Composés de formule I, conformes à la revendication 1, dans lesquels

R1 représente un groupe méthyle,

R2 représente un groupe méthoxy, éthoxy ou propoxy,
R3 représente un groupe méthoxy ou éthoxy, et
R4 représente un groupe phényle portant des substituants R5 et R6, où
R5 représente un atome d'hydrogène, et
R6 représente un groupe de formule -CO-R7 ou -CO-R8, où
R7 représente un groupe hydroxy ou alcoxy en $C_1$-$C_8$, et
R8 représente un groupe de formule -N(R81)R82 où R81 et R82 représentent chacun, indépendamment l'un de l'autre, un atome d'hydrogène ou un groupe alkyle en $C_1$-$C_4$ ou cycloalkyle en $C_5$-$C_7$, ou bien R81 et R82 représentent ensemble, conjointement avec l'atome d'azote auquel ils sont tous les deux liés, un groupe 1-pipéridyle, 1-hexahydroazépinyle ou 4-morpholinyle,

ainsi que les sels de ces composés.

**4.** Composés de formule I, conformes à la revendication 1, dans lesquels

R1 représente un groupe méthyle,
R2 représente un groupe éthoxy,
R3 représente un groupe méthoxy ou éthoxy, et
R4 représente un groupe phényle portant des substituants R5 et R6, où
R5 représente un atome d'hydrogène, et
R6 représente un groupe de formule -CO-R7 ou -CO-R8, où
R7 représente un groupe alcoxy en $C_1$-$C_4$,
et R8 représente un groupe de formule -N(R81)R82 où R81 et R82 représentent chacun, indépendamment l'un de l'autre, un groupe alkyle en $C_1$-$C_4$ ou cycloalkyle en $C_5$-$C_7$, ou bien R81 et R82 représentent ensemble, conjointement avec l'atome d'azote auquel ils sont tous les deux liés, un groupe 1-pipéridyle ou 1-hexahydroazépinyle,

ainsi que les sels de ces composés.

**5.** Composés de formule I, conformes à la revendication 1, dans lesquels

R1 représente un groupe méthyle,
R2 représente un groupe éthoxy ou propoxy,
R3 représente un groupe méthoxy, et
R4 représente un groupe phényle portant des substituants R5 et R6, où
R5 représente un atome d'hydrogène, et
R6 représente un groupe de formule -CO-R8, où
R8 représente un groupe de formule -N(R81)R82, où R81 et R82 représentent chacun, indépendamment l'un de l'autre, un groupe alkyle en $C_1$-$C_4$,

ainsi que les sels de ces composés.

**6.** Composés de formule I, conformes à la revendication 1, dans lesquels les atomes d'hydrogène placés en les positions 4a et 10b se trouvent l'un par rapport à l'autre en situation cis, ainsi que les sels de ces composés.

**7.** Composés de formule I, conformes à la revendication 1, dont les atomes situés en les positions 4a et 10b ont la même configuration absolue que ceux du dichlorhydrate de (-)-cis-4-amino-3-(3-éthoxy-4-méthoxy-phényl)-1-méthylpipéridine présentant un pouvoir rotatoire $[\alpha]_D^{20}$ de-65,5° (c = 1, méthanol), composé utilisable comme produit de départ.

**8.** Composés conformes à la revendication 1, destinés à être utilisés dans le traitement de maladies.

**9.** Médicament contenant un ou plusieurs composés conformes à la revendication 1, ainsi que des adjuvants et/ou véhicules pharmaceutiques usuels.

**10.** Emploi de composés conformes à la revendication 1 pour la fabrication de médicaments destinés au traitement de maladies des voies respiratoires et/ou de dermatoses.